# EUROPEAN PATENT APPLICATION

(11) **EP 1 787 669 A2**
(43) Date of publication of application: **23.05.2007**
(21) Application number: 05767661.1
(22) Date of filing: 19.07.2005
(51) Int. Cl.: A61M 16/00, A61M 1/00

(54) **INTRA-TRACHEAL SPUTUM ASPIRATING APPARATUS**

(30) Priority: 16.07.2004 JP 2004210419
(71) Applicant: Tokunaga, Shuichi, Usa-shi, Ooita 879-0232 (JP)
(72) Inventor: Tokunaga, Shuichi, Usa-shi, Ooita 879-0232 (JP)
(74) Representative: Banzer, Hans-Jörg
(86) International application number: PCT/JP2005/013670
(87) International publication number: WO 2006/009283

(57) **Abstract**

An intra-tracheal sputum aspiration apparatus arranged so that air feeding to and air evacuation from trachea (13) by means of artificial respirator (12) are carried out through tracheal cannula (14) and so that in the event of sputum clogging, tube pump (16A) is operated to thereby attain discharge of sputum through suction tube (15) to the outside of the body by means of a negative pressure produced thereby. By virtue of the employment of the tube pump (16A) as a sputum aspirator (16), the intra-airway pressure by the artificial respirator (12) would have substantially no fluction even when a positive pressure is applied to the lungs. Consequently, sputum disposal can be accomplished while maintaining artificial artificial respiration, so that there can be prevented any secondary infection by infectious agents discharged from sputum collection bottle (17) into the air.

## Description

### FIELD OF THE INVENTION

The present invention relates to an intra-tracheal sputum aspiration apparatus, more in detail, relates to an automatic aspirating technique which is carried out through a hole formed in tracheal by tracheotomy operation.

### BACKGROUND OF THE INVENTION

For the purpose to maintain respiration of a patient, a technique to carry out artificial respiration using a respirator is developed. This technique is to insert a tracheal cannula into incised trachea and to carry out artificial respiration by connecting a respiration tube, which is extended from the respirator, with the tracheal cannula through an adapter. In a case of artificial respiration, sputum accumulated in trachea shuts up respiratory tract. Therefore, treatment of accumulated sputum is a problem.

As an explanatory example of a treating method of sputum, a method that a care giver removes an adaptor from a tracheal cannula, then insert a suction tube into an opening of the tracheal cannula and vacuum off sputum is known. However, by this method, day and night work is required to a care giver and a burden to the care giver is very large.

As a conventional technique to dissolve said problem, for example, an artificial respiration system disclosed in Patent Document 1 is known. In the Patent Document 1, an adapter for artificial respiration and aspiration is equipped with a tracheal cannula, and open and shut manipulation, characterized that when one valve is open another one is shut, is carried out to a couple of valves built in the adapter. By this artificial respiration system, an inner path of the tracheal cannula can be used as a respiration path for ventilation and exhausting and as an aspiration path for sputum. Accordingly, both maintenance of respiration of a patient by a respirator and aspiration of sputum in trachea by a sputum aspirator (intra-tracheal sputum aspiration apparatus) can be possible.

By using an adapter for artificial respiration and aspiration, complicated process of a conventional sputum aspiration method, that is, a care giver removes an adapter of a respirator and stops artificial respiration for short time, and vacuum off sputum by a suction tube, can be omitted.

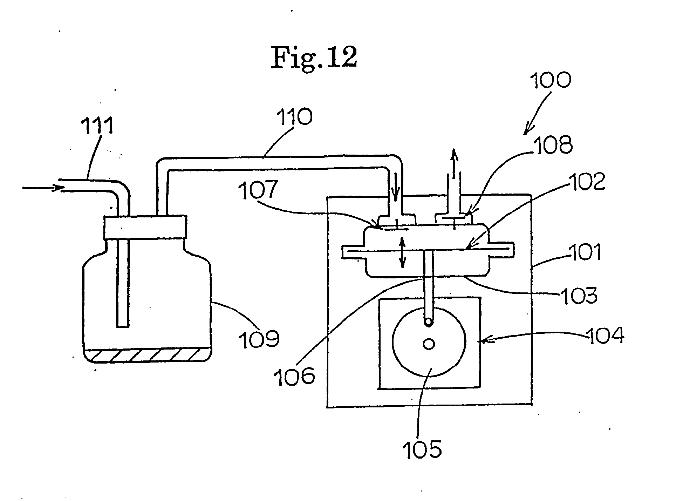

And, as a conventional sputum aspirator for artificial respiration system, for example, as shown in Fig.12, a sputum aspirator 100 using a diaphragm is known.

This sputum aspirator 100 is contained in upper part of an apparatus main body 101, and has a negative pressure casing 103 which is divided to upper and lower parts by a diaphragm 102, a rotation motor 104 contained in lower part of apparatus main body 101, a rotation plate 105 that is rotatable in vertical direction connected with an output shaft of the rotation motor 104, a connecting device 106 whose one end is supported by one part of outermost periphery of the rotation plate 105 and another end is supported by center part of the diaphragm 102 and a ventilation valve 107 and a exhaust valve 108 arranged separately on an upper plate of the negative pressure casing 103. The ventilation valve 107 is connected with a negative pressure tube 110 which is extended from a sputum collection bottle 109. In the meanwhile, the exhaust valve 108 is opened to atmosphere. Further, to the collection bottle 109, a sputum exhaust tube 111 which is connected with sputum exhausting side end of a suction tube (not shown in drawing) is connected.

The rotation plate 105 is rotated by the rotation motor 104 and make descent the connecting device 106 so as to push down center part of the diaphragm 102. Accordingly, upper space of the negative pressure casing 103 becomes negative pressure. As the result, the ventilation valve 107 opens in the condition that the exhaust valve 108 is closed, therefore, the pressure of inner space of the sputum collection bottle 109 becomes negative through a negative pressure tube 110. Consequently, negative pressure acts to a suction tube, which is connected to the sputum collection bottle 109 through 111 and sputum in trachea is sucked into the collection bottle 109.

Patent Document 1; JP2002-219175 publication.

### DISCLOSURE OF THE INVENTION

However, the conventional sputum aspirator 100 has problems mentioned below. That is,
(1) In a case of a grave patient, positive pressure (positive breathing pressure) is always loaded so as to expand a lung. In the conventional sputum aspirator 100, the ventilation valve 107 and the exhaust valve 108 are arranged to a negative pressure casing 103. Therefore, for example, even if a respirator which uses a closed suction system (CCS) that vacuums sputum from a tracheal cannula without remove a respiration tube, when positive pressure acts, there is apprehension that a part of air in trachea is exhausted to outside through the suction tube, the sputum collection bottle 109 and valves 107 and 178 of sputum aspirator 100. By this phenomenon, inner pressure of respiration path of the respirator alters and affect the patient.
(2) In conventional respirator system, even if in a case that uses above mentioned CCS method, valve of an adapter for artificial respiration and aspiration is switched from respiration side to sputum treatment side. Said adapter for artificial respiration and aspiration has complicated valve structural feature characterized that when one valve is open another one is shut. Therefore, respiration of patient stops for short time. That is, it is impossible to treat sputum in trachea with continuing artificial respiration.
(3) When a sputum aspirator 100 with diaphragm is used, even if disinfectant is stored in a sputum collection bottle 109, it is impossible to dip sputum exhaust tube 111 to which a respiration tube is connected and to sterilize sputum without expose sputum to inner space of the sputum collection bottle 109. This is caused by making inside of the sputum collection bottle 109 negative pressure using a negative pressure tube 110 that extends from the collection bottle 109 and aspirating sputum into collection bottle 109 through exhausting opening of suction tube connected with the inner space of the sputum collection bottle 109 by negative pressure power. Consequently, for example, in a case when a patient is a carrier of infectious pathogen such as SAES, there is a possibility to exhaust non-sterilized infectious pathogen to outside through an exhaust valve 108 of the sputum aspirator 100.

The inventors of the present invention continued intensive study and took notice of a tube pump, which is characterized to be possible to transport liquid without using a valve, as a proper sputum aspiration apparatus of CCS type artificial respiration apparatus. This pomp is characterized that a roller equipped to a rotor closes a part of tube and transfer the closing position on a tube to liquid exhausting side continuously so as to extrude liquid contained in the tube continuously. However, suction power of tube pump is weak and flow rate of liquid per unit time is small.

In the meanwhile, sputum formed in trachea of patient, ascends trachea among respiration and exhaust of artificial respiration apparatus and stored gradually at the top of tracheal cannula. Further, at the top of the tracheal cannula an opening for sputum aspiration is formed. Therefore, the inventors of the present invention it is realized that, even if a tube pump whose suction power is small as a sputum aspirator is used, sputum in trachea can be aspirated sufficiently, and accomplished the present invention.

The object of the present invention is to provide a sputum aspirator which can aspirate sputum in trachea continuously and safely without injuring inner surface of trachea, even if in a state that an end part of a suction tube, which is left in trachea, is contacted with inner surface of trachea.

Further, the object of the present invention is to provide a sputum aspirator that can treat sputum characterized that it does not alter inner pressure of trachea of respirator when positive pressure is loaded to lung, can treat sputum with continuing artificial respiration and can prevent secondary infection caused by infectious pathogen existing in air exhausted from a collection bottle.

Furthermore, the object of the present invention is to provide a sputum aspirator that can evade abnormal state during sputum aspiration, can automatically prevent an accident of trachea imperforation, further, can investigate the presence of sputum certainly and quickly.

Still further, the object of the present invention is to provide a sputum aspirator that can notify unusual condition of sputum aspiration to an outsider, accordingly can maintain security of a patient.

### BRIEF ILLUSTRATION OF DRAWING

Figure 1 is the perspective oblique view of an artificial respiration system in which an intra-tracheal sputum aspiration apparatus of Example 1 of the present invention is applied.
Figure 2a is the front magnified view of important part which shows the action of the intra-tracheal sputum aspiration apparatus of Example 1 of the present invention.
Figure 2b is the front magnified view of important part which shows the action of the intra-tracheal sputum aspiration apparatus of Example 1 of the present invention.
Figure 2c is the front magnified view of important part which shows the action of the intra-tracheal sputum aspiration apparatus of Example 1 of the present invention.
Figure 2d is the front magnified view of important part which shows the action of the intra-tracheal sputum aspiration apparatus of Example 1 of the present invention.
Figure 3 is the flow sheet of the control part of an artificial respiration system in which an intra-tracheal sputum aspiration apparatus of Example 1 of the present invention is applied.
Figure 4 is the graph which shows the alteration of aspiration pressure at sputum aspiration action by artificial respiration system in which conventional intra-tracheal sputum aspiration apparatus or an intra-tracheal sputum aspiration apparatus of Example 1 of the present invention is applied.
Figure 5 is the perspective oblique view of an artificial respiration system in which an intra-tracheal sputum aspiration apparatus of Example 2 of the present invention is applied.
Figure 6 is the perspective oblique view of an artificial respiration system in which an intra-tracheal sputum aspiration apparatus of Example 3 of the present invention is applied.
Figure 7 is the perspective oblique view of an intra-tracheal sputum aspiration apparatus of Example 4.
Figure 8 is the front magnified view of important part which shows the aspiration state of membrane of sputum by an intra-tracheal sputum aspiration apparatus of Example 4.
Figure 9 is the front magnified view of important part which shows the aspiration state of membrane of sputum by an intra-tracheal sputum aspiration apparatus of Example 4.
Figure 10 is the perspective oblique view of an intra-tracheal sputum aspiration apparatus of Example 5.
Figure 11 is the flow sheet of the control part of an intra-tracheal sputum aspiration apparatus of Example 5.
Figure 12 is the front view showing the use of conventional intra-tracheal sputum aspiration apparatus.

Illustrations of marks used in drawings are as follows.
10, 10A, 10B: artificial respiration system 11: respiration tube
12: respirator 13: trachea 14: tracheal cannula 15: suction tube
16, 160, 160A: sputum aspiration apparatus (intra-tracheal sputum aspiration apparatus) 16A: tube pump 16B: support pump
18: control part 31: pressure sensor 51: rotor 51a: push roller
52: rotating mean 53: pressure guide 53a: tube pressing surface

### PREFERRED EMBODIMENT OF THE INVENTION

The invention of claim 1 is an intra-tracheal sputum aspiration apparatus comprising, a suction tube that aspirates sputum formed in trachea, wherein one end part of the suction tube is left in trachea, and a pump mean connected with said suction tube and generates negative pressure to aspirate said sputum, wherein said pump mean is a tube pump comprising, a rotor to which plural pressure rollers, which press and block a part of transformable elastic suction tube, are installed to have nodules at the outermost periphery direction of the rotor separately, a pressure guide whose surface facing to said rotor is a tube pressing surface, which curves parallel with outermost periphery of the rotor, and a rotating mean to rotate said rotor.

By the invention of claim 1, sputum stored in trachea can be sucked off to outside through a suction tube by negative pressure (suction power) generated in the pump by moving the tube pump.

Specifically, suction tube is placed between outermost periphery of the rotor and tube pressing surface of a pressure guide, then the rotor is rotated to sputum exhausting direction by a rotating mean. Accordingly, a pressure roller is rotated toward sputum exhausting direction along with the tube pressing surface of the pressure guide. At this time, a part of suction tube is blocked between tube pressing surface of side of the pressure guide and the pressure roller. The blocked position transfers to exhausting direction continuously and sputum stored in suction tube is extruded toward exhausting direction. Accordingly, negative pressure is generated at aspiration side of the suction tube and sputum in trachea is sucked into the suction tube. Consequently, sputum is exhausted from the suction tube.

Further, a tube pump is the pump which sucks continuously at low speed, quantitatively (for example, 10-100cc/sec.) and at high pressure. Consequently, in the state that sputum does not close the suction tube, vacuum pressure in tube does not rise, and by the state that the tube is clogged by sputum, vacuum pressure rises. Accordingly, even if the tube pump is worked always, there is no problem in respiration. Further, even if in the state that the end part of the suction tube which is left in trachea is contacted with inner wall of trachea, there is no apprehension to injure inner wall of trachea by a part of inner wall of trachea is sucked into the suction tube. Therefore, it is possible to aspirate sputum continuously and safely.

The material, diameter and length of the suction tube are not restricted as long as it is a tube of elastic and transformation free. As a starting material of a suction tube, soft polyethylene, soft polypropylene, soft polyurethane, soft silicone, soft polyetheretherketone or soft vinyl chloride can be used.

The end part of a suction tube can be inserted directly in trachea or can be inserted through tracheal cannula. As a suction tube, a separable type separated to a part, which is left in trachea and a part which extends to a pump, can be used.

As the subject to be aspirated by the suction tube, sputum ca be mentioned. Further, other secretion such as spittle can be mentioned. A pump mean is an apparatus to aspirate sputum stored in trachea through a suction tube by negative pressure by negative pressure generated in inside.

Kinds of starting material and diameter of a pressure roller, a rotor and a pressure guide are not restricted. As a pressure guide, for example, board material or block material can be used. For example, as a pressure guide, gutter shape type can be used.

As a rotating mean, for example, electric motor or air motor can be used.

The invention mentioned in claim 2, is relating to a intra-tracheal sputum aspiration apparatus comprising, a respirator to which a respiration tube for respiration and exhausting is connected and an intra-tracheal sputum aspiration apparatus connected with said respiration tube, which is used for artificial respiration system providing a tracheal cannula to be inserted into trachea of patient, said intra-tracheal sputum aspiration apparatus aspirates sputum through a suction tube by a pump mean in a state that the respiration tube is connected to a tracheal cannula, wherein said pump mean is a tube pump comprising, a rotor to which plural pressure rollers, which press and block a part of transformable elastic suction tube, are installed to have nodules at the outermost periphery direction of the rotor separately, a pressure guide whose surface facing to said rotor is a tube pressing surface, which curves parallel with outermost periphery of the rotor,
and a rotating mean to rotate said rotor.

According to the invention of claim 2, air supply and exhausting to trachea of patient by a respirator is carried out through a tracheal cannula. While, in a case that sputum is stored in trachea, sputum can be sucked off through the suction tube by negative pressure generated in the pump by driving the tube pump.

Specifically, suction tube is placed between outermost periphery of the rotor and tube pressing surface of a pressure guide, then the rotor is rotated to sputum exhausting direction by a rotating mean. Accordingly, a pressure roller is rotated toward sputum exhausting direction along with the tube pressing surface of the pressure guide. At this time, a part of suction tube is blocked between tube pressing surface of side of the pressure guide and the pressure roller. The blocked position transfers to exhausting direction continuously and sputum stored in suction tube is extruded toward exhausting direction. Accordingly, negative pressure is generated at aspiration side of the suction tube and sputum in trachea is sucked into the suction tube. Consequently, sputum is exhausted from the suction tube.

Further, a tube pump is the pump which sucks continuously at low speed, quantitatively and at high pressure. Consequently, in the state that sputum does not close the suction tube, vacuum pressure in tube does not rise, and by the state that the tube is clogged by sputum, vacuum pressure rises. Accordingly, even if the tube pump is worked always, there is no problem in respiration. Further, even if in the state that the end part of the suction tube which is left in trachea is contacted with inner wall of trachea, there is no apprehension to injure inner wall of trachea caused by vacuum pressure. Therefore, it is possible to aspirate sputum continuously and safely. Of cause, it is possible to operate a tube pump by every certain time using a timer controller.

By using a tube pump, in a case when a patient is a severe patient, for example, who always needs to keep lung at positive pressure, there is no apprehension that a part of air in trachea is exhausted to outside from respiration valve or exhaust valve caused by positive pressure, which is usual in a case of conventional diaphragm type intra-tracheal sputum aspiration apparatus. Accordingly, when it is used at positive pressure condition, inner trachea pressure of a respirator does not alter, and aspiration of sputum does not affect the respiration of a patient. Further, since a tube pump is used, for example, in a case that a sputum collection bottle contains sterilization agent, a sputum exhausting tube, which is connected to a suction tube, is dipped directly in the sterilization agent and is possible to sterilize sputum without expose to inner space of the sputum collection bottle. Consequently, it becomes possible to protect from secondary infection caused by infectious pathogen existing in air exhausted from a collection bottle. This is caused by fact that sputum is aspirated in a condition that a part of a suction tube is always blocked by a pressing roller. In other words, by aspirating sputum in a closed circuit, it becomes possible not to expose sputum to inner space of the sputum collection bottle.

A respirator is an apparatus which repeats air supply and air exhaust each other by certain time interval to trachea, for the purpose to maintain respiration of a patient. And, structural feature of a respirator is not restricted.

Tracheal cannula is a short tube part which is inserted into trachea through a hole which is formed to trachea by tracheotomy. Numbers of inner path of tracheal cannula can be only one or can be plural. Further, at the outermost periphery surface of end part of trachea side of tracheal cannula, an extendable cuff by air depositing and drawing can be equipped for the purpose to block up the gap between trachea and tracheal cannula.

Wording that "fixing of a suction tube to tracheal cannula" means not only to fix a suction tube to tracheal cannula in one body, but also to fix a suction tube to tracheal cannula admitting freely equipping and removing.

A suction tube can be fixed to inner surface of path wall of tracheal cannula or to the outermost surface of tracheal cannula. Further a suction tube can be a suction tube which is separated admitting freely equipping and removing to a part fixed to the suction tube and a part extended to a pump mean.

The invention mentioned in claim 3 is relating to an intra-tracheal sputum aspiration apparatus of claim 1 or claim 2, wherein the suction tube is provided with a pressure sensor which detects inner pressure of the suction tube, said suction tube is connected with a support pump which sucks off the stored sputum in trachea by stronger suction power than that of the tube pump, and when the pressure sensor detects the state that the inner pressure of said suction tube is higher than the inner pressure of normal sputum aspiration, enhance the suction power or drive said support pump.

According to the invention of claim 3, during sputum aspiration, for example, large quantity of sputum is formed or high viscosity sputum is sucked, suction power becomes larger than that of normal sputum aspiration. And when this abnormal state is detected by a pressure sensor, operation command is sent from control part to a tube pump or a support pump and suction power of the tube pump is enhanced or the support pump driven. Consequently, such an abnormal state is evaded and accident by tracheal obturation can be automatically protected. Further, since inner pressure of a suction tube can be detected by a pressure sensor, existence of sputum can be detected certainly and promptly.

Furthermore, by prescribing inner pressure value (prescript value) of suction tube that indicates to change from a suction tube to a high power support pump, proper aspiration treatment of sputum according to amount of sputum or viscosity of sputum becomes possible.

During aspiration process by a support pump, aspiration by tube pump can be continued or can be stopped.

Position to detect inner pressure (suction pressure) of suction tube is not restricted. For example, it can be in the suction tube or it is possible to equip a branch tube and to equip a pressure sensor at the top of the branch tube so as to detect inner pressure.

A support pump can be connected with a suction tube trough a branch tube. And, at the connecting part of the branch tube and the suction tube, a changeover valve (relief valve) which changes suction direction of sputum to the support pump (branch tube side) can be provided.

In the case to switch from aspiration by the tube pump to aspiration by the support pump, value for switching (prescript value) is not restricted. For example, value of inner pressure of a suction tube at the maximum output power can be used. Further, prescript value can be changed considering height or weight of a patient. Specifically, is from 5 to 15KPa.

Kinds of a support pump is not restricted. For example, a diaphragm sputum aspiration pump can be used.

Invention of claim 4 relates to an intra-tracheal sputum aspiration apparatus according to claim 1 or claim 2, wherein said intra-tracheal sputum aspiration apparatus has an alarm mean which sounds warning, said suction tube provides a pressure sensor, which detects inner pressure of the suction tube, said pressure sensor detects elevation of inner pressure of the suction tube, and when said detecting state is over the prescript time, sounds warning by said alarm mean.

According to the invention of claim 4, state that the elevation of inner pressure of the suction tube progresses the prescript time, for example, obsturation state of the suction tube or the state of low suction speed in the tube caused by too high viscosity of sputum are detected and sounds warning alarm so as to inform the abnormal state to an outsider. Accordingly, security of a patient can be maintained. To an alarm mean, a circuit to generate warning alarm and a speaker are equipped.

### EXAMPLES

### Examples of the present invention will be specifically illustrated.

### Example 1

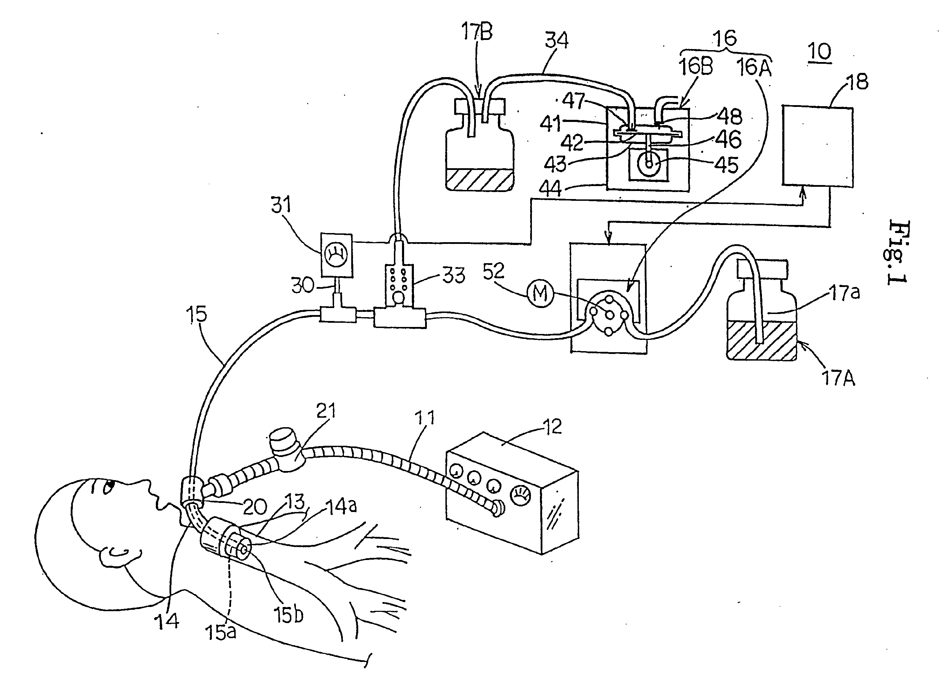

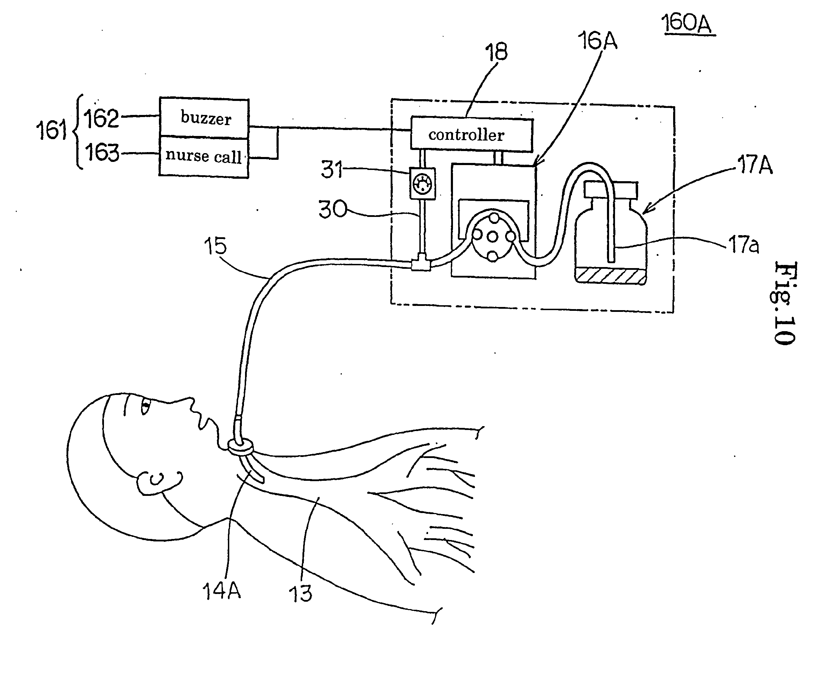

In Fig.1, 10 indicates an artificial respiration system which uses an intra-tracheal sputum aspiration apparatus of Example 1. This artificial respiration system provides a respirator 12 to which a respiration tube 11 for air supply and for exhausting are connected, a tracheal cannula 14 connected with a respiration tube 11, which is to be inserted into trachea 13 of a patient, a sputum aspiration apparatus (intra-tracheal sputum aspiration apparatus) 16 to which a suction tube (suction catheter) 15 connected with the respiration tube 11 and to suck out sputum stored in trachea 13 is connected, two sputum collection bottles 17A and 17B and a control part 18 which controls suction pressure of sputum by the sputum aspiration apparatus 16 and controls ON·OFF of the respirator 12 by a sequencer.

The respirator 12 is a device characterized to drive an installed compressor by every certain interval so as to maintain respiration of patient. The respiration tube 11 is connected with air supplying and exhausting opening of the respirator 12 and with a mount (joint part) 20 set to the base point of tracheal cannula. On the way of the respiration tube 11, an expiration valve 21 which carries out exhausting is provided. The tracheal cannula 14 is a plastic tube having curved shape like as J, which is to be inserted into a hole formed in trachea 13 of patient by trachea incision. Inner diameter of the tracheal cannula 14 is 9mm and the end part is opened in trachea.

In a tube of the tracheal cannula 14 an edge part of the suction tube 15 which is extended from the sputum aspiration apparatus 16 through above mentioned mount 20. The inserting position of end part of the suction tube 15 is same position as to end of the tracheal cannula 14. Outer diameter of the suction tube 15 is 4mm. Therefore, at the inner path of the tracheal cannula 14, large respiration path 14a of 51mm² cross section is maintained. Accordingly, a patient can breathe easily.

Further, in the suction tube 15, a suction path of sputum 15a is formed. Still further, at the end part of the suction tube 15 a suction hole of sputum 15b is formed. The position to form the suction hole of sputum 15b is at the end of the suction tube 15.

At the upper stream (tracheal cannula 14) side from the middle of length direction of the suction tube 15, a short first branch tube 30, which is used to detect suction pressure by the sputum aspiration apparatus 16 is connected. The end part (upper end part) of the first branch tube 30 is connected to a pressure sensor 31. By the pressure sensor 31, inner pressure (suction pressure of sputum in a tube) of the suction tube 15 can be measured.

Furthermore, in the suction tube 15, closely to the downstream of the first branch tube 30, a long second branch tube 32 is connected. At the base part (connecting part with the suction tube 15) of the second branch tube 32, a relief valve 3, which opens by transferring a steel ball (valve body) against spring power of a coil spring when inner pressure of the suction tube 15 exceeds prescript value (for example 10KPa), is provided. The end part of the suction tube 15 is inserted in inner space of the closed up sputum collection bottles 17B. And, the inner space of the sputum collection bottles 17B and a suction valve of a support pump 16B, which will be illustrated below, are connected by a negative pressure tube 34.

The sputum aspiration apparatus 16 is an apparatus to suck off sputum stored in trachea 13 by negative pressure generated in inside through the suction tube 15 and to gather it in the sputum collection bottle 17A or in the sputum collection bottle 17B. Details of the sputum aspiration apparatus 16 will be illustrated below.

In the control part 18, above mentioned sequencer and a timer (not indicated by drawing) are equipped.

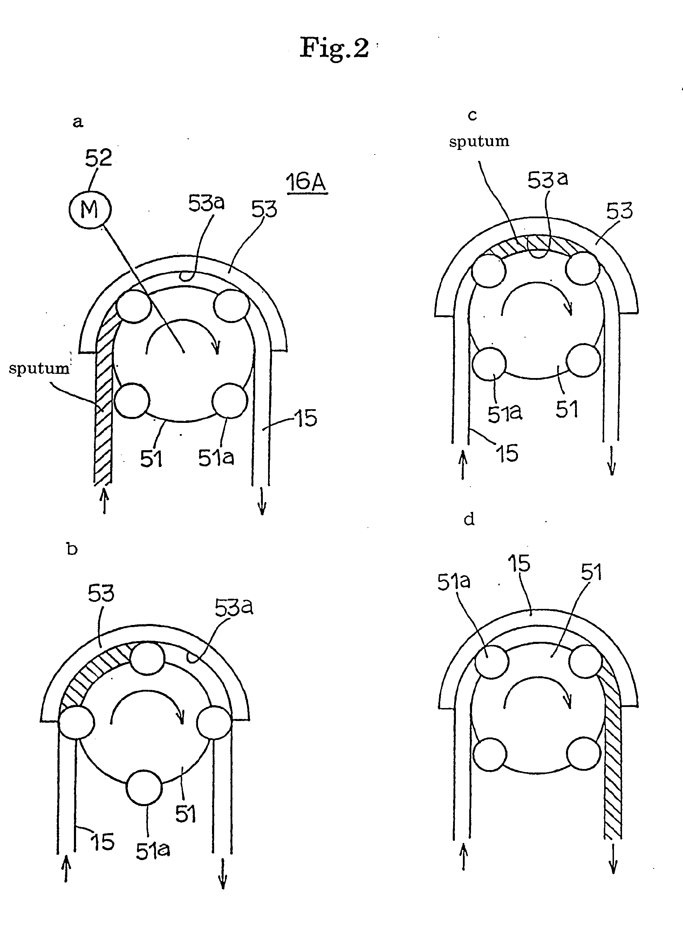

The sputum aspiration apparatus 16 will be illustrated more in detail referring to Figs 1 and 2.

The sputum aspiration apparatus 16 provides a tube pump (pump mean) 16A and a support pump 16B.

The tube pump 16A provides a rotor 51 to which four pressure rollers 51a, which press and close a part of a suction tube 15, are projected at the outermost periphery direction separately, and a pressure guide 53 provided so as to have prescript distance from the outermost surface of the rotor 51, whose facing surface to said rotor 51 is to be a tube pressing surface 53a, which curves parallel with outermost periphery of the rotor 51 and a rotating mean 52 to rotate said rotor 51.

The rotating mean 52 is an electric motor and it rotates the rotor 51 along with the tube pressing surface 53a of the pressure guide 53. Transferring amount (transferring speed) of sputum by the tube pump 16A is 100cc/sec.

A support pump 16B sucks off sputum stored in trachea 13 by higher suction power than the maximum suction power of the tube pump 16A. Specifically, a diaphragm pump is used. That is, this support pump 16B is contained in upper part of apparatus main body, and has a negative pressure casing 43 which is divided to upper and lower parts by a diaphragm 42, a rotation motor 44 contained in lower part of apparatus main body 41, a rotation plate 45 rotatable in vertical direction, a connecting device 46 connected to an output shaft of the rotation motor 44, wherein whose one end is supported by one part of outermost periphery of the rotation plate 45 and another end is supported by center part of the diaphragm 42 and a ventilation valve 47 and a exhaust valve 48 arranged separately on an upper plate of the negative pressure casing 43. The ventilation valve 47 is connected with a negative pressure tube 34, which is extended from the sputum collection bottle 17B. In the meanwhile, the exhaust valve 48 is opened to atmosphere.

The rotation plate 45 is rotated by the rotation motor 44 and make descent the connecting device 46 so as to push down center part of the diaphragm 42. Accordingly, upper space of the negative pressure casing 43 becomes negative pressure. As the result, the ventilation valve 47 opens in the condition that the exhaust valve 48 is closed, therefore, the pressure of inner space of the sputum collection bottle 17B becomes negative through a negative pressure tube 34. Consequently, large negative pressure acts to a suction tube 15, which is connected to the sputum collection bottle 17B through second branch tube 32 and sputum in trachea is sucked into the sputum collection bottle 17B.

As the next, control of sputum aspiration by above mentioned control part 18 will be illustrated. At the normal sputum aspiration, the tube pump 16A is moved by prescript pressure value (normal suction pressure at sputum aspiration), and when detected value detected by a pressure sensor 31 becomes higher than the prescript value, operation command is sent to the tube pump 16A and to enhance the output of pump for prescript time, or operation command is sent to the support pump 16B and drives the support pump 16B for prescript time.

That is, during normal operation of the sputum aspiration apparatus 16, the tube pump 16A is driven always, and when an abnormal state, such as a case that sputum clogs in the suction tube 16, is caused, enhance output of the tube pump 16A or drive the support pump 16B.

The function of an artificial respiration system 10 of Example 1 of the present invention will be illustrated as follows.

As shown in Fig.1, respiration of a patient, namely, air supply and exhausting to trachea 13 of the patient using a respirator 12 is carried out by a respiration path 14a in tracheal cannula 14 through a respiration tube 11 and a mount 20.

In the meanwhile, when sputum clogs in trachea 13, by driving the tube pump 16A at all times, sputum is sucked off from the body through the suction tube 15 by negative pressure power generated in the pump.

Specifically, in the state that the suction tube 15 is arranged between outermost surface of a rotor 51 and a tube pressing surface 53a of a pressure guide 53, the rotor 51 is rotated by a rotating mean 52 to sputum exhausting side (Fig.2a). Consequently, the pressure roller 51a rotates to sputum exhausting side along with the tube pressing surface 53a of the pressure guide 53. At this point, a part of the suction tube 15 is blocked between the tube pressing surface 53a side of the pressing guide 53 and the pressure roller 51a. The blocked position transfers continuously to sputum exhausting side along with the rotation of the pressure roller 51a (Fig.2b, Fig.2c).

Consequently, closing position on the suction tube 15 moves gradually toward sputum exhausting side, and sputum in the suction tube 15 is transferred toward sputum exhausting side. Finally, sputum is exhausted from the suction tube 15 (Fig.2d). Thus, a sputum aspiration apparatus of simple and low cost structure can be obtained. Accordingly, at the suction side of the suction tube 15, negative pressure power is generated and sputum in trachea 13 is sucked into suction tube 15. At this time, an inner path of trachea cannula 14 and the respirator 12 is always connected. Therefore, even if during sputum aspiration, patient's respiration can be maintained too.

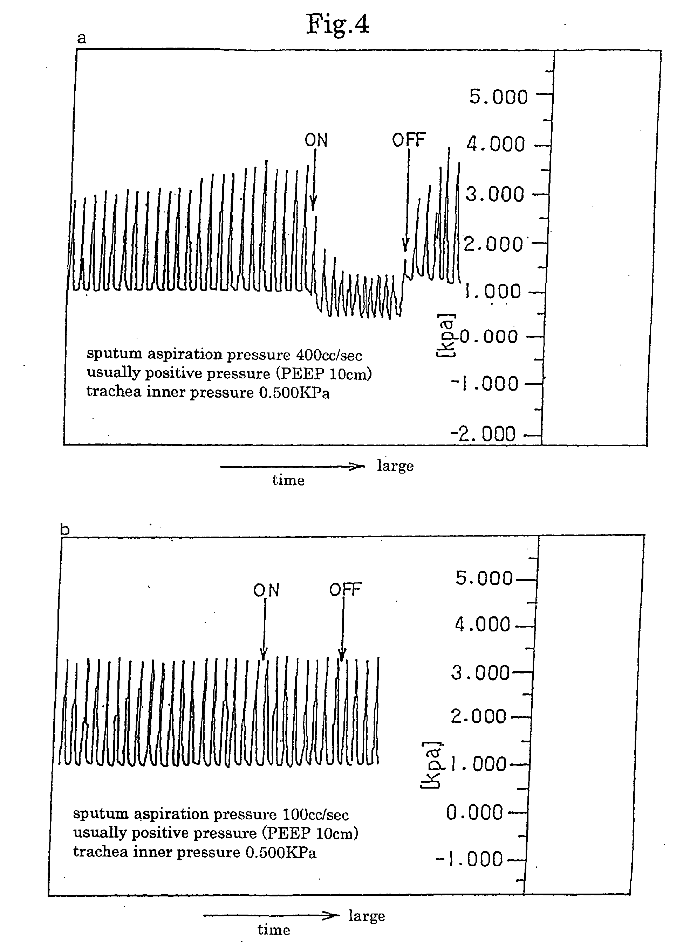

Further, by use of a tube pump 16A, for example, even if in a case when a patient is a severe patient, for example, who always needs to keep his lung at positive pressure, there is no apprehension that a part of air in trachea is exhausted to outside from respiration valve or exhaust valve caused by positive pressure, which is usual in a case of conventional diaphragm type intra-tracheal sputum aspiration apparatus. Accordingly, when it is used at positive pressure condition, when compared with a case of diaphragm type intra-tracheal sputum aspiration apparatus (graph of Fig.4a), inner trachea pressure of a respirator 12 does not alter, and aspiration of sputum does not affect the respiration of a patient (graph of Fig.4b). In graphs of Fig.4a and Fig.4b, ON indicates starting of sputum aspiration and OFF indicates end of sputum aspiration.

Furthermore, a tube pump 16A is used as a pump mean, in a case that a sputum collection bottle contains sterilization agent, a top edge part 17a of a suction tube 15, is dipped directly in the sterilization agent and is possible to sterilize sputum without expose to inner space of the sputum collection bottle 17A. Consequently, it becomes possible to protect from secondary infection caused by infectious pathogen existing in air exhausted from a collection bottle 17A. This is caused by fact that sputum is aspirated in a condition that a part of the suction tube is always blocked by a pressing roller. Accordingly, it becomes possible to treat sputum without exposing it to inner space of the sputum collection bottle 17A.

Further, a tube pump 16A is a pump that sucks continuously at low speed, quantitatively (100cc/sec.) and at high pressure. Consequently, even if the tube pump 16A is driven all the time, there is no interruption to respiration. Further, in the state that sputum does not close a sputum aspiration opening 15b of suction tube 15, vacuum pressure in tube does not rise, and by the state that the suction tube15 is clogged by sputum, vacuum pressure rises for the first time. Consequently, even if in the state that the end part of the suction tube 15 which is left in trachea 13 is contacted with inner wall of trachea, there is no apprehension to injure inner wall of trachea by a part of inner wall of trachea is sucked into the suction tube 15. And, it is possible to detect the presence of sputum certainly and promptly by a pressure sensor 31. e, it is possible to aspirate sputum continuously and safely.

In a case when large quantity of sputum is formed, which can not be treated by a tube pump 16A, or a suction tube 15 is clogged by high viscosity sputum, during sputum aspiration, suction power becomes larger than that of normal sputum aspiration. And when this abnormal state is detected by a pressure sensor 31 and the detected value reaches to the prescript value, operation command is sent from a control part 18 to the tube pump 16A or to a support pump 16B. Consequently, such an abnormal state is evaded and accident by tracheal obturation can be automatically protected. In a case when the support pump 16B is driven, large negative pressure is loaded to the suction tube 15 which is connected with a sputum collection bottle 17B, through second branch tube 32 and a relief valve 33, and sputum in trachea 14 is sucked into the sputum collection bottle 17B.

Thus, in a case that the suction pressure of sputum becomes larger than the prescript value (normal value), selection either to enhance the suction power of the tube pump 16A or to drive the support pump 16B should be previously decided by a medical doctor by operating a power input key. Of cause it is possible to enhance the suction power of the tube pump 16A or to drive the support pump 16B by every certain time interval by a timer control.

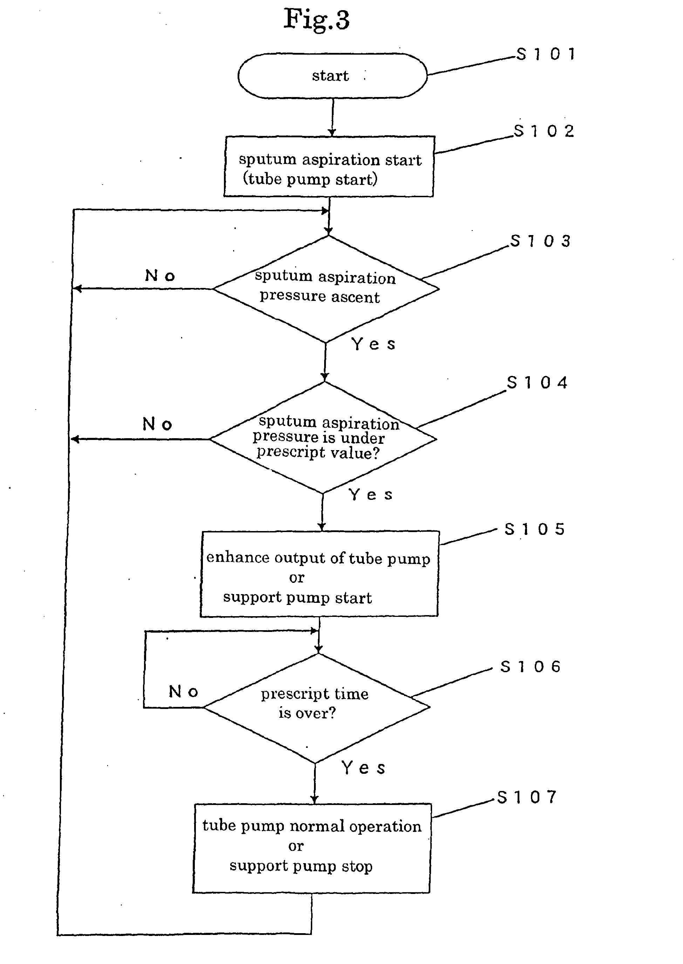

The suction operation of sputum by a sputum aspiration apparatus 16 will be illustrated as follows, referring to a flow sheet of Fig.3.

By turning a switch of electric power, a control circuit is started (S101) and a tube pump 16A is driven so that the suction of sputum is started (S102). Then the ascent of suction pressure of the tube pump 16A is confirmed (S103). If the suction pressure is ascent, advance to S104, and if not, go back to S103. At S104, it is detected whether the suction pressure of the tube pump 16A reaches to the prescript value or not. If the value is less than the prescript value, advance to S105. At this point, suction amount of sputum is enhanced by increasing rotating number of a motor of the tube pump 16A per unit time, or drive a support pump 16B. Then by a timer, whether 15 seconds is past or not is detected (S106), and if not past, continue the suction of sputum by high power by the tube pump 16A or the support pump 16B. After 15 seconds laps, the timer is off, and back the suction pressure of the tube pump 16A to the value of normal operation, or stop the driving of the support pump 16B and switch to the normal operation (S107). And, back to S103.

As another method for sputum aspiration control by a sputum aspiration apparatus 16, for example, in ascent state of suction pressure, output of a tube pump 16A is gradually ascent until the suction pressure reaches to the prescript switching value, and when the suction pressure is ascent to the level that the tube pump 16A can not manage, pump can be changed to a support pump 16B. Accordingly, it becomes possible to carry out suction treatment of sputum according to the amount of sputum or viscosity of sputum.

Further, as a different method for sputum aspiration control by a sputum aspiration apparatus 16, for example, it is possible to use a method to set low prescript value and high prescript value. Specifically, for example, maintain the output (suction power) of the tube pump 16A as it is until reaches to the low prescript value. And, between low prescript value and high prescript value, adjust the suction power of the tube pump 16A along with the detected value. And, when reach to the high prescript value, operate the support pump 16B. By controlling as above, it is possible to carry out suction treatment of sputum according to the amount of sputum or viscosity of sputum.

### Example 2

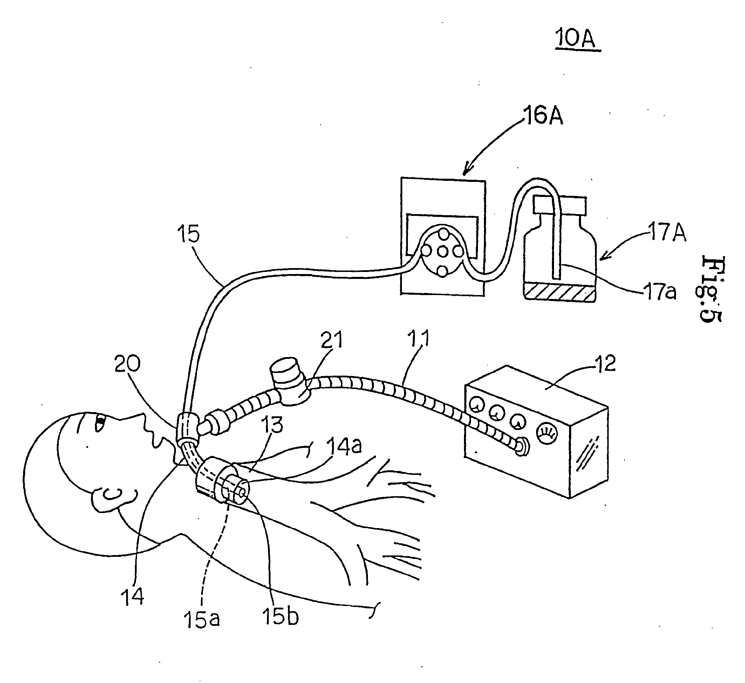

The intra-tracheal sputum aspiration apparatus of Example 2 of the present invention will be illustrated referring to Fig.5. As shown in Fig.5, an artificial respiration system 10A which uses the intra-tracheal sputum aspiration apparatus of Example 2 is not an example that control the suction of sputum in response to alteration of inner pressure of a suction tube 15 based on the detected signal of a pressure sensor 31 like as to Example 1, but an example to use only tube pump 16A and sucks sputum continuously by low speed, quantitatively (100cc/sec.) and at high pressure.

Therefore, in Example 2, support pump 16B, sputum collection bottle 17B, control part 18, first branch tube 30, second branch tube 32, relief valve 33 and negative pressure tube 34 are omitted. Therefore, suction control of sputum using a pressure sensor 31 can not be carried out, but the numbers of parts of an artificial respiration system 10A are reduced and can be fabricated by lower cost.

Since other construction, function and effect are almost same as to that of Example 1, illustration is cut out.

### Example 3

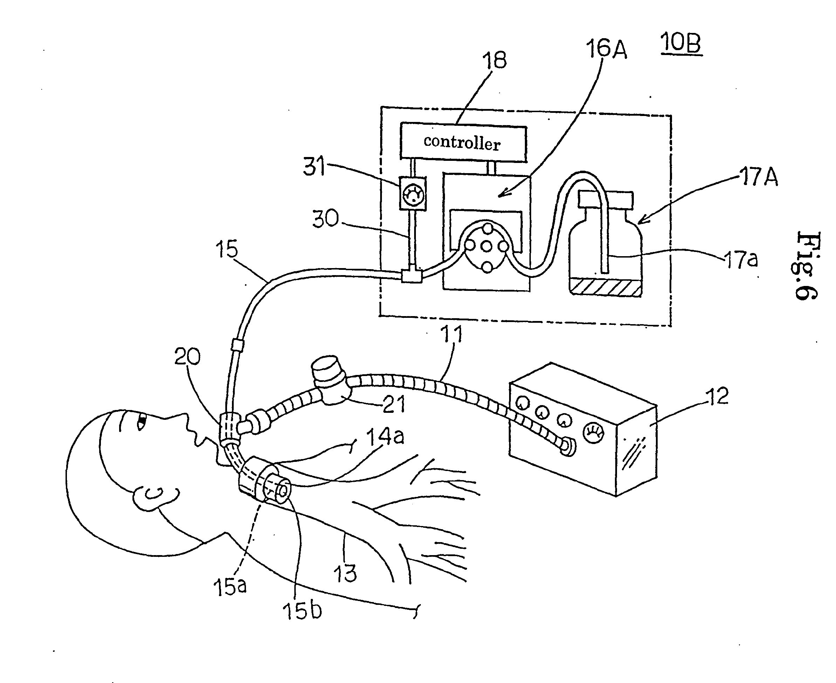

The intra-tracheal sputum aspiration apparatus of Example 3 of the present invention will be illustrated referring to Fig.6.

As shown in Fig.6, an artificial respiration system 10B, which uses the intra-tracheal sputum aspiration apparatus of Example 3, is an example that control the output of a tube pump 16A in response to alteration of inner pressure of a suction tube 15 based on the detected signal of a pressure sensor 31.

Specifically, when suction pressure of sputum is ascent, it is detected by a pressure sensor 31, and a control part 18 ascends the output of the tube pump 16A. On the contrary, when suction pressure of sputum is dropped, the drop is detected by the pressure sensor 31, and the control part 18 makes drop the output of the tube pump 16A.

Accordingly, the numbers of parts of an artificial respiration system 10A are reduced compared with Example 1 and can be fabricated by lower cost maintaining the suction control of sputum using the pressure sensor 31 possible.

Since other construction, function and effect are almost same as to that of Example 1, illustration is cut out.

### Example 4

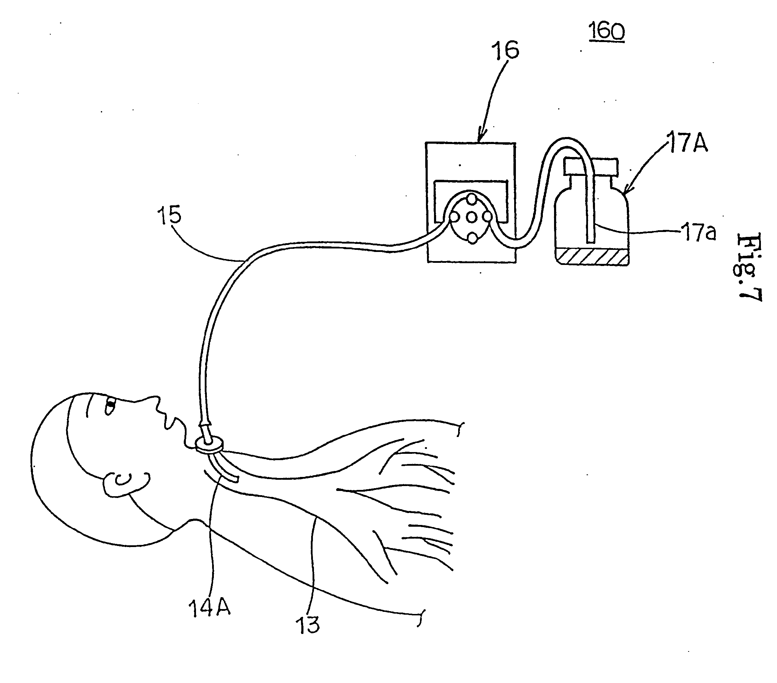

The intra-tracheal sputum aspiration apparatus of Example 4 of the present invention will be illustrated referring to Figs.7-9.

As shown in Fig.7, an artificial respiration system 160, which uses the intra-tracheal sputum aspiration apparatus of Example 4, is an example that a respirator 12 is omitted from Example 2 and uses single tube structure trachea cannula 14A instead of double tube structure trachea cannula 14, and a suction tube 15 is connected to the trachea cannula 14A directly. Therefore, a respiration tube 11, a mount 20 and an expiration valve 21 are omitted.

Accordingly, it becomes possible to suck off sputum continuously, even if in the state that the end part of the suction tube 15, which is left in trachea 13, is contacted with inner wall of trachea, there is no apprehension to injure inner wall of trachea by a simple and low price apparatus.

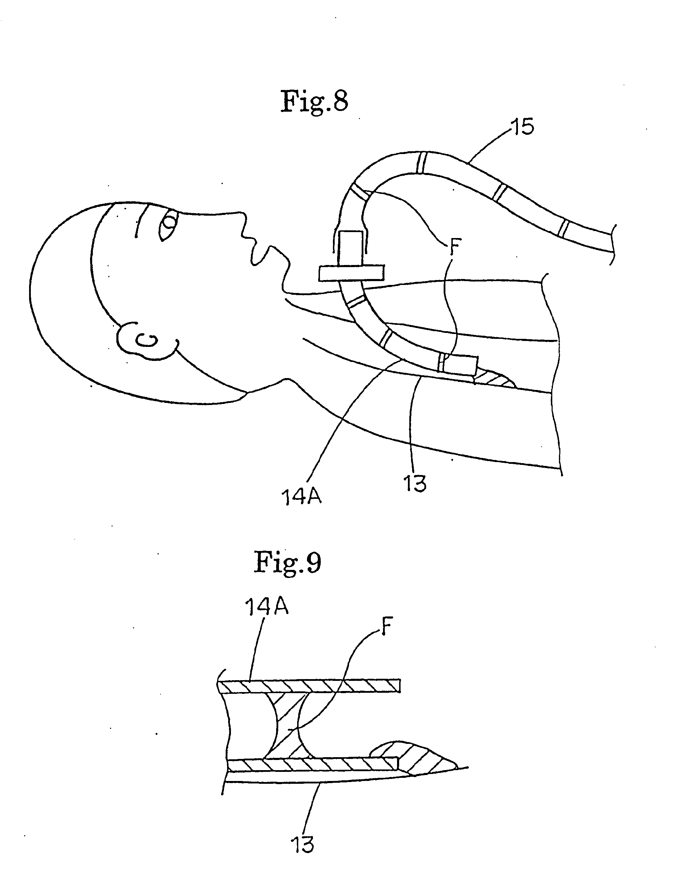

Further, as shown in Fig.8 and Fig.9, at the suction of sputum, some times film of sputum F is formed intermittently in the suction tube 15 and trachea cannula 14A caused by surface tension. Said film is easily to break when small amount of sputum is formed by usual in 13. When these film F is tried to removed by a conventional sputum aspirator using a diaphragm, the film is broken by its high suction pressure and can not be sucked off by good condition. However, by a tube pump 16A, which makes possible low speed suction of sputum, it become possible to suck off sputum continuously without breaking these films F. Said effect of suction and removal of said film F is not limited to Example 4, but covers whole this invention that uses a tube pump.

Since other construction, function and effect are almost same as to that of Example 1, illustration is cut out.

### Example 5

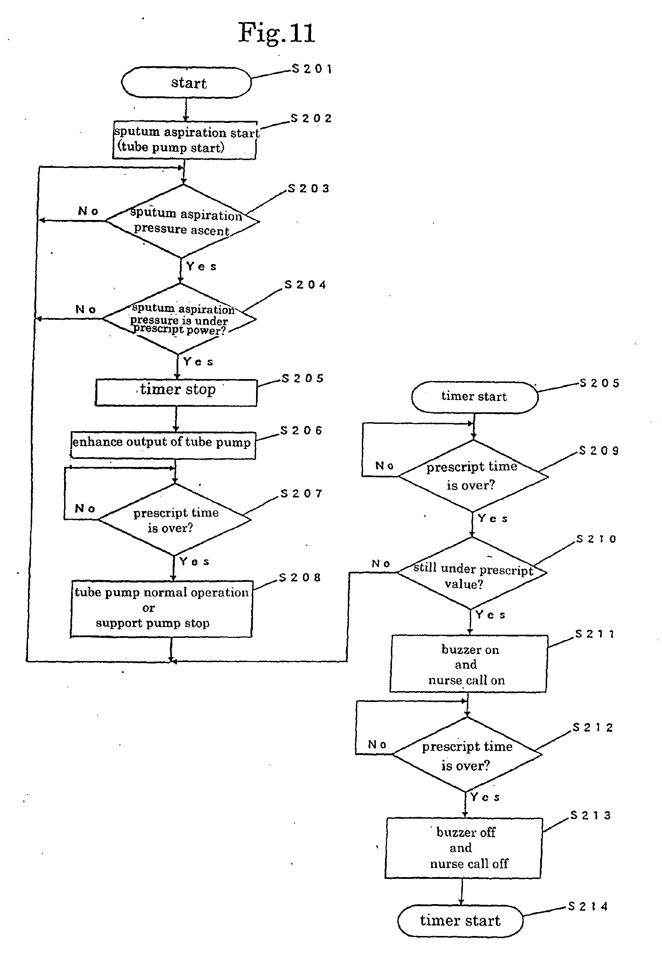

The intra-tracheal sputum aspiration apparatus of Example 5 of the present invention will be illustrated referring to Fig.10 and Fig.11.

As shown in Fig.10, an artificial respiration system 160A of Example 5 of the present invention is an example which makes possible in Example 4 to sound warning alarm and nurse call by an alarm mean 161 which is connected electrically to a control part 18, when output control of the tube pump 16A based on detecting signal from a pressure sensor 31 and abnormal state of high sputum suction pressure is continued for long time.

Specifically, when the suction pressure of sputum is ascent it is detected by a pressure sensor 31, and a control part 18 ascends the output of the tube pump 16A. On the contrary, when suction pressure of sputum is dropped, the drop is detected by the pressure sensor 31, and the control part 18 makes drop the output of the tube pump 16A.

Accordingly, the numbers of parts of an artificial respiration system 10A are reduced compared with Example 1 and can be fabricated by lower cost maintaining the possibility of suction control of sputum using the pressure sensor 31.

Further, when a state that ascent of inner pressure of a suction tube 15 continues for prescribed time, for example, clogged up state of the suction tube 15 or state of low suction speed in the tube caused by too high viscosity of sputum are detected, warning alarm is sounded from an alarm mean 161 and nurse call sign is sent to a nurse station. That is, in the alarm mean 161, a buzzer 162 and a nurse call sending part 163 are equipped. Consequently, this abnormal state can be informed to a medical doctor or an outsider, or to a nurse waiting in the nurse station certainly and instantly. Thereby, safety of a patient can be maintained.

Suction operation of sputum by intra-tracheal sputum aspiration apparatus 160A will be illustrated referring to a flow sheet of Fig.11.

Turning a switch of electric source and start a control circuit (S201), a tube pump 16A begins to work and starts suction of sputum (202). Then judges whether suction pressure of the tube pump 16A is ascent (S203). If the suction pressure is ascent, go to S, and if not, back to S203. At S204, whether suction pressure of the tube pump 16A is less than prescribed value or not is judged. If the suction pressure is less than the prescribed value, first timer and second timer are started respectively (S205). Timer control circuit that uses second timer will be illustrated later. After that, output of the tube pump 16A is increased so as to enhance suction amount of sputum (S206). Then, judges by first timer whether 15 seconds is passed or not (S207), and if not, continue the suction of sputum by tube pump 16A whose output is increased. After 15 seconds passed, turn off the first timer and suction pressure is back to normal operation level (S208). Then, back to S203.

Timer control circuit will be illustrated here. After the second timer is started (S205), whether prescribed time (several seconds to several minutes) is passed or not is judged by the second timer (S209). After the prescribed time is passed, whether the state of less than prescribed value is continued or not is judged (S210). If not, the second timer is turned off and go back to S203, and if yes, it is judged that abnormal state is caused and warning alarm is sounded by buzzer 162 and nurse call sign is sent from nurse call sending part 163 (S211). Then by the second timer, whether prescribed seconds are passed after warning alarm and nurse call are sounded, is judged (S212). After passed, warning alarm and nurse call are stopped (S213) and stop the timer (S214).

Other constructions, function and effect are same as to Example 1, therefore, illustration is omitted.

### POSSIBILITY FOR INDUSTRIAL USE

According to the invention mentioned in claim 1, since a tube pump is used as a pump mean, even if in a case that the tube pump is operated, there is no interruption for respiration of a patient. Further, even if in the state that the end part of the suction tube which is left in trachea is contacted with inner wall of trachea, there is no apprehension to injure inner wall of trachea by a part of inner wall of trachea is sucked into the suction tube. Therefore, it is possible to aspirate sputum continuously and safely.

According to the invention mentioned in claim 2, since a tube pump is used as a pump mean, in a case when positive pressure is loaded to lung, inner pressure of trachea of respirator does not alter and can treat sputum with continuing artificial respiration and can prevent secondary infection caused by infectious pathogen existing in air exhausted from a collection bottle. Further, even if in the state that the end part of the suction tube which is left in trachea is contacted with inner wall of trachea, there is no apprehension to injure inner wall of trachea by a part of inner wall of trachea is sucked into the suction tube. Therefore, it is possible to aspirate sputum continuously and safely.

In particular, according to the invention mentioned in claim 3, during sputum aspiration, for example, suction power becomes larger than that of normal sputum aspiration, and when this abnormal state is detected by a pressure sensor, suction power of the tube pump is enhanced or the support pump driven. Consequently, such an abnormal state is evaded and accident by tracheal obturation can be automatically protected. Further, since inner pressure of a suction tube can be detected by a pressure sensor, existence of sputum can be detected certainly and promptly.

According to the invention mentioned in claim 4, the state that ascent of inner pressure of a suction tube continues prescribed time is detected by a pressure sensor and sounds warning alarm and the abnormal state can be informed to the outsider. Accordingly, the safety of the patient can be maintained.

## Claims

1. An intra-tracheal sputum aspiration apparatus comprising, a suction tube that aspirates sputum formed in trachea, wherein one end part of the suction tube is left in trachea, and a pump mean connected with said suction tube and generates negative pressure to aspirate said sputum,
wherein said pump mean is a tube pump comprising,
a rotor to which plural pressure rollers, which press and block a part of transformable elastic suction tube, are installed to have nodules at the outermost periphery direction of the rotor separately,
a pressure guide whose surface facing to said rotor is a tube pressing surface, which curves parallel with outermost periphery of the rotor,
and a rotating mean to rotate said rotor.

2. An intra-tracheal sputum aspiration apparatus comprising, a respirator to which a respiration tube for respiration and exhausting is connected and an intra-tracheal sputum aspiration apparatus connected with said respiration tube, which is used for artificial respiration system providing a tracheal cannula to be inserted into trachea of patient, said intra-tracheal sputum aspiration apparatus aspirates sputum through a suction tube by a pump mean in a state that the respiration tube is connected to a tracheal cannula,
wherein said pump mean is a tube pump comprising,
a rotor to which plural pressure rollers, which press and block a part of transformable elastic suction tube, are installed to have nodules at the outermost periphery direction of the rotor separately,
a pressure guide whose surface facing to said rotor is a tube pressing surface, which curves parallel with outermost periphery of the rotor,
and a rotating mean to rotate said rotor.

3. The intra-tracheal sputum aspiration apparatus of claim 1 or claim 2, wherein the suction tube is provided with a pressure sensor which detects inner pressure of the suction tube, said suction tube is connected with a support pump which sucks off the stored sputum in trachea by stronger suction power than that of the tube pump, and when the pressure sensor detects the state that the inner pressure of said suction tube is higher than the inner pressure of normal sputum aspiration, enhance the suction power or drive said support pump.

4. The intra-tracheal sputum aspiration apparatus according to claim 1 or claim 2, wherein said intra-tracheal sputum aspiration apparatus has an alarm mean which sounds warning, said suction tube provides a pressure sensor, which detects inner pressure of the suction tube, said pressure sensor detects ascent of inner pressure of the suction tube, and when said detecting state is over the prescript time, sounds warning by said alarm mean.
